# EUROPEAN PATENT SPECIFICATION

(11) **EP 2 125 767 B1**
(45) Date of publication and mention of the grant of the patent: **26.02.2014**
(21) Application number: 07847567.0
(22) Date of filing: 30.11.2007
(51) Int. Cl.: C07D 309/04, C07D 309/10, C07D 309/18, A61K 8/49, A23L 1/226, A61Q 13/00

(54) **PYRAN DERIVATIVES, PROCESS OF PREPARATION AND USE THEREOF IN PERFUMERY AND FLAVOURING**
PYRAN-DERIVATE, VERFAHREN ZU IHRER HERSTELLUNG UND IHRE VERWENDUNG IN DER PARFÜMERIE UND AROMATISIERUNG
DÉRIVÉS DE PYRANNE, PROCÉDÉ DE PRÉPARATION ET UTILISATION DE CEUX-CI DANS LA PARFUMERIE ET L'ASSAISONNEMENT

(30) Priority: 01.12.2006 EP 06291857; 01.12.2006 US 861966 P
(43) Date of publication of application: 02.12.2009
(73) Proprietor: V. Mane Fils, 06620 Bar sur Loup (FR)
(72) Inventor: PLESSIS, Caroline, 06620 Bar sur Loup (FR); MANE, Jean, 06130 Grasse (FR)
(74) Representative: Cabinet Plasseraud
(86) International application number: PCT/EP2007/063051
(87) International publication number: WO 2008/065181

(56) References cited:
- EP-A- 0 189 144
- EP-B1- 0 746 552
- FR-A- 1 013 862
- US-A- 4 963 285
- DATABASE CAPLUS CHEMICAL ABSTRACTS SERVICE, COLUMBUS, OHIO, US; XP002430734 retrieved from STN Database accession no. 1977:535063 -& JP 52 007911 A (OTSUKA PHARMACEUTICAL CO LTD) 21 January 1977 (1977-01-21) -& DATABASE WPI Week 197709, Derwent Publications Ltd., London, GB; AN 1977-15605Y, XP002430741 & JP 52 007911 A (OTSUKA PHARM CO LTD) 21 January 1977
- K. MIKAMI ET AL: J. CHEM. SOC., CHEM. COMM., no. 24, 1993, pages 1843-1844, XP000944733
- E. MONTAUDON ET AL: J. HETEROCYCLIC CHEM., vol. 28, no. 2, 1991, pages 459-463, XP002472220

## Description

The present invention relates to the field of fragrances and flavours. More particularly, the invention relates to pyran derivatives, the process of preparation thereof, and their use in the field of perfumery and flavouring.

The terms "fragrance" and "fragrant", as used herein, are used interchangeably whenever a compound or a mixture of compounds is referred to, which is intended to pleasantly stimulate the sense of smell.

The terms "flavour", and "flavouring", as used herein, are used interchangeably whenever a compound or a mixture of compounds is referred to, which is intended to stimulate the sense of taste and smell. Also in the meaning of the invention, the term flavouring relates to the flavouring of any liquid or solid, human or animal, in particular of drinks, dairy products, ice creams, soups, sauces, dips, dishes, meat products, culinary assistances, salted biscuits or snacks. It also means the flavouring of beers, wines and tobaccos.

The term "organoleptic compound", such as for example fragrances and flavours, as used herein, refers to compounds of the invention which stimulate the sense of smell or taste, and are thus perceived as having a characteristic odour and/or flavour.

By the term "masking" is meant reducing or eliminating malodour or bad flavour perception generated by one or more molecules entering in the composition of a product.

The term "isomer", in the present invention, means molecules having the same chemical formula, which means same number and types of atoms, but in which the atoms are arranged differently. The term "isomer" includes structural isomers, geometric isomers, optical isomers and stereoisomers.

The term "alkyl" or "alkyl group", in the present invention, means any linear or branched saturated hydrocarbon chain, having preferably 1, 2, 3, 4 or 5 carbon atoms and herein referred to as C₁₋₅ alkyl group, such as for example methyl, ethyl, propyl, isopropyl, butyl, t-butyl, pentyl.

The term "alkenyl" or "alkenyl group", in the present invention, means any linear or branched mono or poly unsaturated hydrocarbon chain, having preferably 2, 3, 4 or 5 carbon atoms and herein referred to as C₂₋₅ alkenyl group, such as for example ethenyl, propenyl, butenyl, or pentenyl.

The term "cycloalkyl" or "cycloalkyl group", in the present invention, means any cyclic saturated hydrocarbon chain having preferably 5, 6 or 7 carbon atoms, such as for example cyclopentyl, cyclohexyl and cycloheptyl, substituted or not by an alkyl or alkenyl group as described above.

The term "cycloalkenyl" or "cycloalkenyl group", in the present invention, means any cyclic mono or poly unsaturated hydrocarbon chain having preferably 5, 6 or 7 carbon atoms, such as for example cyclopentenyl, cyclohexenyl and cycloheptenyl, substituted or not by an alkyl or alkenyl as described above.

The term "cycloalkylacetaldehyde", in the present invention, represents an acetaldehyde substituted by a cycloalkyl group.

The term "cycloalkenylacetaldehyde", in the present invention, represents an acetaldehyde substituted by a cycloalkenyl group.

Tetrahydropyran and dihydropyran derivatives belong to an important class of fragrant ingredients and a large work has already been done to prepare known compounds, such as for example Rose Oxide and similar derivatives, from linear or branched alkyl and alkenyl aldehydes, as described in US 3,681,263 and in WO 04/009749, or from benzylic aldehydes as described in CH 655 932.

Similarly, esters or ethers of pyranols have been found of interest in the aromatic industry as shown in US 4,963,285, US 4,962,090.

JP25-7911 discloses ethers, including 2-(1-cyclohexylethyl)-tetrahydro-2H-pyran, useful as perfume.

EP0189144 discloses a pyran derivative mixture wherein the 2,4-dimethyl-3-cyclohexenyl moiety is directly attached position 2 of the pyran ring, and the use thereof in perfume compositions.

EP0746552 discloses tetrahydropyran derivatives wherein the cyclopentyl or cyclopentenyl moiety is directly attached to position 5 of the pyran ring, and the use thereof as odorants

US4963285 discloses pyran-4-ol derivatives having a C2-C4 straight chain or branched chain alkyl or alkenyl group in position 2 of the pyran ring and the use thereof for enhancing the aroma of consumable materials.

US 2,452,977 relates to a process for the production of cyclic compounds; according to this process, an unsaturated alcohol is reacted with an aldehyde to produce a cyclic compound having an oxygen atom within the ring. Some resulting compounds are pyrans.

The need of new compounds is of great importance for the development of the flavour and fragrance industry, which recently had to face stricter international regulatory requirements about the use of certain materials, as well as environmental concerns and customer demands for improved performance. Being able of manufacturing new fragrant compounds is therefore a challenge.

When exploring further the pyran derivatives in order to identify new fragrant compounds, the Applicant focused on lateral cyclic chains, more specifically on cycloalkylacetaldehydes and cycloalkenylacetaldehydes as starting materials.

None of the above-cited document mentions nor suggests a possible use of cycloalkylacetaldehyde , or cycloalkenylacetaldehyde to prepare pyran derivatives, and of course, as the resulting pyran derivatives are new, there is no prior art information regarding their possible olfactive or organoleptic properties.

This invention thus relates to pyran derivatives of general formula (I) 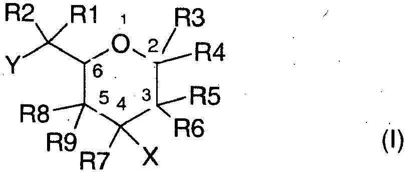 wherein
Y = 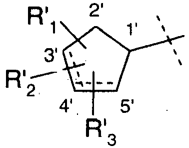 wherein R'1, R'2 and R'3 are each independently a hydrogen atom or a methyl group or an ethyl group, at least one of R'1, R'2 and R'3 being a methyl or an ethyl group,
   wherein the dotted lines represent a double bond and is present or not.
   - R1, R2, R3, R4 are, each independently, a hydrogen atom or a linear or branched C₁₋₅ alkyl or C₂₋₅ alkenyl group, and
   - X is present or absent,
   - when X is present,
      o R5, R6, R7, R8, R9 are all present, and X is a hydrogen atom or an OZ group, wherein Z is a hydrogen atom or a R10 group or a C(O)R10 group
   - when X is absent, a double bond involving the carbon atom at the 4 position is present and
      o R7, R8 and R9 are present, and one of R5 or R6 is present and the other one is absent, or
      o R5, R6, and R7 are present, and one of R8 or R9 is present and the other one is absent, or
      o R7 is a =C(R11) (R12) group and R5, R6, R8, R9 are present,
   - and when they are present, each of R5-R12 group is, independently, a hydrogen atom, or a linear or branched C₁₋₅ alkyl or C₂₋₅ alkenyl group.

In a specific embodiment, the invention relates to pyran derivatives of general formula (I) as described above, wherein
- Y is one of a (Y1), (Y2), (Y3) or (Y4) group 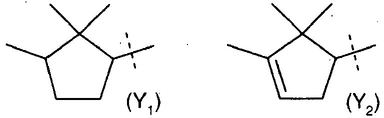 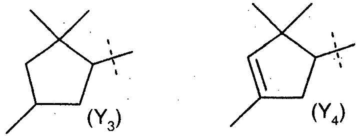
- R1, R2, R3, R4 are, each independently, a hydrogen atom, a linear or branched C₁₋₅ alkyl or C₂₋₅ alkenyl group, and
- X is present or absent,
- when X is present,
   o R5, R6, R7, R8, R9 are all present, and X is a hydrogen atom or an OZ group, wherein Z is a hydrogen atom or a R10 group or a C(O)R10 group
- when X is absent, a double bond involving the carbon atom at the 4 position is present and
   o R7, R8 and R9 are present, and one of R5 or R6 is present and the other one is absent, or
   o R5, R6, and R7 are present, and one of R8 or R9 is present and the other one is absent, or
   o R7 is a = C(R11) (R12) group and R5, R6, R8, R9 are present,
- and when they are present, each of R5-R12 group is, independently, a hydrogen atom, a linear or branched C₁₋₅ alkyl or C₂₋₅ alkenyl group.

In another embodiment, preferred pyran derivatives of general formula (I) as described above, are those wherein
- Y is a 5-, 6- or 7-membered ring, preferably a 5-membered ring, optionally methyl or ethyl mono- or polysubstituted, and optionally unsaturated, or Y is a (Y1), (Y2), (Y3), (Y4) group,
- one of R3 or R4 is an hydrogen atom, and the other one is a methyl group
- R7 is a =CH₂ group and X is absent; or
- R7 is a methyl group and X is present or absent ; when R7 is a methyl group and X is present, X is an hydrogen atom or an OZ group, wherein Z is an hydrogen atom or a R10 group or a C(O)R10 group; when R7 is a methyl group and X is absent, a double bond involving the carbon atom at the 4 position is present and
   o R8 and R9 are present, and one of R5 or R6 is present and the other one is absent, or
   o R5, R6, are present, and one of R8 or R9 is present and the other one is absent,
- R1,R2 and R10 are each independently a hydrogen atom, or a linear or branched C₁₋₅ alkyl or C₂₋₅ alkenyl group
- When present, R5, R6, R8, R9 are each a hydrogen atom.

In still another embodiment, preferred pyran derivatives of general formula (I) as described above, are selected in the group comprising 4-methylene-2-(2,2,3-trimethyl-cyclopent-3-enylmethyl)-tetrahydropyran, 4-methyl-6-(2,2,3-trimethyl-cyclopent-3-enylmethyl)-3,6-dihydro-2H-pyran, 4-methyl-2-(2,2,3-trimethyl-cyclopent-3-enylmethyl)-3,6-dihydro-2H-pyran, 4-methyl-2-(2,2,3-trimethyl-cyclopentylmethyl)-tetrahydropyran, 4-methylene-2-(2,2,3-trimethyl-cyclopentylmethyl)-tetrahydropyran, 4-methyl-6-(2,2,3-trimethyl-cyclopentylmethyl)-3,6-dihydro-2H-pyran, 4-methyl-2-(2,2,3-trimethyl-cyclopentylmethyl)-3,6-dihydro-2H-pyran, 4-methyl-4-(3-methyl-but-3-enyloxy)-2-(2,2,3-trimethyl-cyclo-pentylmethyl)-tetrahydropyran.

According to another embodiment, pyran derivatives of the invention are of general formula (I) wherein
- Y is a 5-, 6-, or 7-membered ring, optionally methyl or ethyl mono or polysubstituted, and saturated; or
- Y is a 5- or 7-membered ring, optionally methyl or ethyl mono or polysubstituted, and polyunsaturated; or
- Y is a 5- or 6-membered ring, optionally methyl or ethyl mono or polysubstituted, mono or di-unsaturated, provided that the double bond is not in position 1', or
- Y is a 7-membered ring, optionally methyl or ethyl mono or polysubstituted, mono-unsaturated, or
- R1, R2, R3, R4 are, each independently, a hydrogen atom or a linear or branched C₁₋₅ alkyl or C₂₋₅ alkenyl group, and
- X is present or absent,
- when X is present,
   o R5, R6, R7, R8, R9 are all present, and X is a hydrogen atom or an OZ group, wherein Z is a hydrogen atom or a R10 group or a C(O)R10 group
- when X is absent, a double bond involving the carbon atom at the 4 position is present and
   o R7, R8 and R9 are present, and one of R5 or R6 is present and the other one is absent, or
   o R5, R6, and R7 are present, and one of R8 or R9 is present and the other one is absent, or
   o R7 is a =C(R₁₁)(R12) group and R5, R6, R8, R9 are present,
- and when they are present, each of R5-R12 group is, independently, a hydrogen atom, or a linear or branched C₁₋₅ alkyl or C₂₋₅ alkenyl group.

In a second aspect, the invention relates to a process for the preparation of the pyran derivatives of the invention, said process being characterized in that a compound of general formula (A) 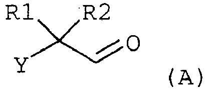 wherein
- Y is a 5-, 6- or 7-membered ring, preferably a 5-membered ring, optionally methyl or ethyl mono- or polysubstituted, and optionally unsaturated,
- R1 and R2 are, each independently, a hydrogen atom or a linear or branched C₁₋₅ alkyl or C₂₋₅ alkenyl group,
is reacted, in presence of an acid, with a compound of general formula (B) 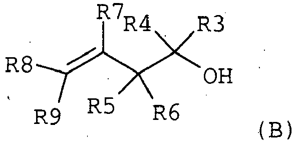 wherein
R3, R4, R5, R6, R7, R8 and R9 are, each independently, a hydrogen atom or a linear or branched C₁₋₅ alkyl group, or a linear or branched C₂₋₅ alkenyl group.

According to an embodiment of the invention, the process further comprises a step to convert the aldehyde (A) into an acetal of general formula (C) 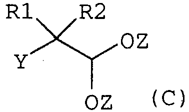 wherein
- Y is a 5-, 6- or 7-membered ring, preferably a 5-membered ring, optionally methyl or ethyl mono- or polysubstituted, and optionally unsaturated,
- R1, R2, are, each independently, a hydrogen atom, a linear or branched C₁₋₅ alkyl or C₂₋₅ alkenyl group,
- Z is a R10 group wherein R10 is a linear or branched C₁₋₅ alkyl or C₂₋₅ alkenyl group,
prior to the reaction with the alcohol (B).

According to an embodiment, (A) and/or (B) and/or (C) is used as an optically pure isomer.

In another embodiment, the invention relates to the process as described above, wherein compound (A) or compound (C) is reacted, in presence of an acid, with compound (B), and from the resulting mixture, the different compounds of formula (I), wherein X is OZ, Z being a hydrogen atom or a linear or branched C₁₋₅ alkyl or C₂₋₅ alkenyl group, or wherein X is absent, are separated.

According to another embodiment of the invention, compound (A) is reacted with compound (B), in presence of an acid, and from the resulting mixture, the compounds of formula (I), wherein X is OH, are separated from the compounds of formula (I) wherein X is absent, and then subjected to an esterification reaction, in order to obtain compounds of formula (I) wherein X is OC(O)R10.
The esterification reaction may be performed by usual procedures known by one skilled in the art.

According to another embodiment of the invention, compound (A) is reacted with compound (B), in presence of an acid, and from the resulting mixture, the compounds of formula (I), wherein X is OH, are separated from the compounds of formula (I), wherein X is absent and then subjected to an alkylation reaction in order to obtain compounds of formula (I) wherein X is OZ, Z being a linear or branched C₁₋₅ alkyl or C₂₋₅ alkenyl group.
The alkylation reaction may be performed by usual procedures known by one skilled in the art.

In another particular embodiment, the invention relates to the process as described above, wherein compound (A) is reacted with compound (B) and the resulting mixture of pyran derivatives is subjected to a dehydration reaction in presence of an acid, said process resulting in the manufacturing of compounds of formula (I) wherein X is absent, and a double bond at the 4 position is present.

Separation steps may be performed by any techniques known from one skilled in the art.

Another aspect of the invention is a composition comprising at least one compound of formula (II), and/or at least one compound of general formula (III) and/or at least one compound of general formula (IV), and optionally (V) and/or (VI) 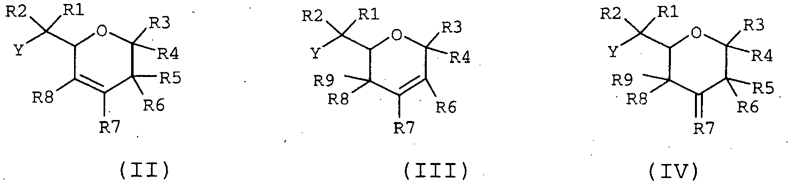 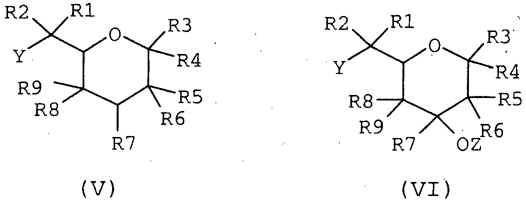 wherein
- Y is a 5-, 6- or 7-membered ring, preferably a 5-membered ring, optionally methyl or ethyl mono- or polysubstituted, and optionally unsaturated,
- R1, R2, R3, R4, R5, R6, R7, R8, R9 are, each independently, a hydrogen atom, a linear or branched C₁₋₅ alkyl or C₂₋₅ alkenyl group,
- Z is a hydrogen atom or a linear or branched C₁₋₅ alkyl or C₂₋₅ alkenyl group or a group C(O)R10 wherein R10 is a linear or branched C₁₋₅ alkyl or C₂₋₅ alkenyl group.

This invention relates to the compounds of formula (I), as described above, but also to any of their various isomers.

In a last aspect, the invention relates to the use of the compounds of formula (I) as described above.

In a first embodiment, the invention relates to the use of at least one compound or of the composition as described above, in the perfumery field for the preparation of perfumed bases and concentrates, fragrances, perfumes and similar products; topic compositions; cosmetic compositions such as for example face and body creams, cleansers, facial treatments, talc powders, hair oils, shampoos, hair lotions, bath oils and salts, shower and bath gels, soaps, body anti-perspirants and deodorizers, pre-shave, shaving and post-shave creams and lotions, creams, toothpastes, mouth baths, pomades; cleaning products, such as for example softeners, detergents, air deodorizers and household cleaning supplies. Therefore, the invention also relates to a fragrant composition including at least one compound of formula (I) or one or more isomers of a compound of formula (I).

In a second embodiment, the invention relates to the use of at least one compound or of the composition as described above, as flavouring agents for the preparation of flavouring compositions or articles, such as for example drinks, dairy products, ice creams, soups, sauces, dips, dishes, meat products, culinary assistances, salted biscuits or snacks and also beers, wines and tobaccos. Therefore, the invention also relates to a flavoured composition including at least one compound of formula (I) or one or more isomers of a compound of formula (I).

In a third embodiment, the invention relates to the use of the compounds or composition as described above, as masking agents of odours and/or flavours, and to any pharmaceutical, cosmetic or food composition containing at least one compound of formula (I) or one or more isomers of a compound of formula (I). Therefore, this invention also relates to any composition comprising at least one compound of formula (I), as herein described, in combination with any suitable excipient, especially pharmaceutical or cosmetic or dietary excipient.

The compounds of the invention may be used alone or in combination with other perfuming or flavouring ingredients, solvents, additives or fixatives, commonly used and that the man skilled in the art is able to choose in regard of the desired effect and the nature of the product to perfume or flavour. This invention includes, for example, any composition comprising one or more isomers of a compound of formula (I). According to a preferred embodiment, this invention relates to a composition comprising at least two or three isomers of a compound of formula (I).

In another embodiment, in the uses as described above, the compounds of the invention are used in a concentration comprised in a range from 0.001% to 99% in weight, in particular from 0.1% to 50% in weight, more particularly from 0.1% to 30% in weight. It is known by the man skilled in the art that these values depend on the nature of the composition/article to be perfumed and/or flavoured, the desired intensity of the perfume and/or flavour, and the nature of the other ingredients present in said composition or article. According to a preferred embodiment, the compounds of the invention are used in an olfactory effective amount. By olfactory effective amount is meant a level or amount of fragrant/flavouring compound in a material at which the incorporated compound exhibits a sensory effect.

Following examples detail the preparation processes of the compounds of the invention and their use. These examples are presented with an only goal of illustration.

### Example 1: Preparation of

### 4-Methylene-2-(2,2,3-trimethyl-cyclopent-3-enylmethyl)-tetrahydro-pyran (A)

### 4-Methyl-6-(2,2,3-trimethyl-cyclopent-3-enylmethyl)-3,6-dihydro-2H-pyran (B)

### 4-Methyl-2-(2,2,3-trimethyl-cyclopent-3-enylmethyl)-3,6-dihydro-2H-pyran (C)

A mixture of 1 equivalent of campholenaldehyde and 2 equivalents of 3-methyl-3-buten-1-ol in toluene, with catalytic amount of PTSA, is heated under reflux to remove the formed water. After completion of the reaction (3 hours), the mixture is cooled down and diluted with toluene. After washing with a saturated bicarbonate solution and brine, the organic phase is dried over MgSO₄, filtered and the solvents are evaporated to afford a crude oil.

The distilled product (82°C/ 0.8 torr) consists in a mixture of isomers (A), (B) and (C) in a 27:16:57 ratio.

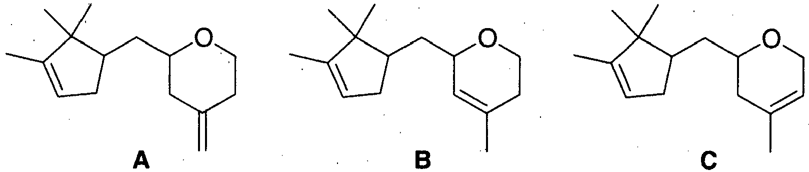

Its odour is powerful, hesperidic (bitter orange, petitgrain), green (galbanum, peas).

The different isomers are obtained separately by a fine fractionation distillation with a packed column under partial reflux.
Each isomer is a mixture of 2 diastereoisomers.

¹H-NMR (CDCl₃, 200MHz) : common protons δ (ppm) 0,75 (s, 3H), 0,97 (s, 3H), 1.25-2.5 (m, 7H), 1.59 (s, 3H), 5,22 (m, 1H).

### Characteristic protons:

**(A)** δ (ppm) 1,25-2,5 (m, 2H), 3.2-3.7 (m, 3H), 4.7 (m, 2H).
**(B)** δ (ppm) 1.68 (s, 3H), 3.9-4.2 (m, 3H), 5,32 (m, 1H).
**(C)** δ (ppm) 1.68 (s, 3H), 3.2-3.7 (m, 1H), 3.9-4.2 (m, 2H), 5.4 (m, 1H).

IR (film, cm⁻¹): 796m, 1014m, 1095m, 1122s, 1138m, 1360m, 1382m, 1437m, 1445m, 1463m, 2864s, 2930s, 2955s.
MS [e/m (%)]: 220(M+, 12), 149(5), 135(4), 119(8), 108(100), 93(59), 79(19), 67(35), 53(20), 41(48).

### Example 2: Preparation of 4-methyl-2-(2,2,3-trimethyl-cyclopentylmethyl)-tetrahydro-pyran

A mixture of isomers, prepared in example 1, is treated under a normal pressure of hydrogen in ethanol with catalytic amount of palladium on charcoal (5%) to give the desired tetrahydropyran. The crude product obtained after filtration of the reaction mixture on Clarcel® is purified by distillation (88°C/ 0.7 torr). It consists in four couples of diastereoisomers (2 major and 2 minor, the ratio between them is 36:42:10:12).
Its odour is bitter orange, woody, a bit dusty/musky. ¹H-NMR (CDCl₃, 200MHz): common protons δ (ppm) 0.46 & 0.48 (s, 3H), 0.65-0.87 (m, 6H), 0.90-1.30 (m, 4H), 1.30-2.20 (m, 9H), 3.10-3.75 (m, 2H).
Major isomers: *Characteristic protons* δ (ppm) 0.90 (d, J=6.1Hz) & 0.91 (d, J=6.2Hz) : 3H ; 3.85-4.0 (m, 1H).
Minor isomers: *Characteristic protons* δ (ppm) 1.01 (d, 1H, J=7.1Hz) & 1.02 (d, 1H, J=7.1Hz) : 3H ; 3.10-3.75 (m, 1H). ¹³C-NMR (CDCl₃, 60MHz):
Major isomers: 13.9 & 14.26, 22.36 & 24.40, 25.37 & 25.46, 28.11 & 28.39, 30.14 & 30.26; 30.27 & 30.41, 34.75 & 34.82, 37.40 & 37.51, 40.21 & 41.87, 42.03 & 42.35, 44.90 & 44.96, 46.11 & 47.01, 67.99 & 68.12, 76.37 & 77.18.
Minor isomers (selected data): 13.90 & 14.26, 18.72 & 19.18, 24.78 & 24.94, 28.0 & 28.32, 32.4 & 32.62, 35.44 & 35.96, 37.19 & 38.82, 44.87, 47.4.
IR (film, cm⁻¹): 1095s, 1177m, 1366m, 1374m, 1386m, 1456m, 1466m, 2838m, 2869s, 2926s, 2952s.
MS [e/m (%)]: 224 (M+, 1), 99(100), 55(18), 43(19), 41(17).

### Example 3: Preparation of

### 4-Methylene-2-(2,2,3-trimethyl-cyclopentylmethyl)-tetrahydro-pyran (A)

### 4-Methyl-6-(2,2,3-trimethyl-cyclopentylmethyl)-3,6-dihydro-2H-pyran (B)

### 4-Methyl-2-(2,2,3-trimethyl-cyclopentylmethyl)-3,6-dihydro-2H-pyran (C)

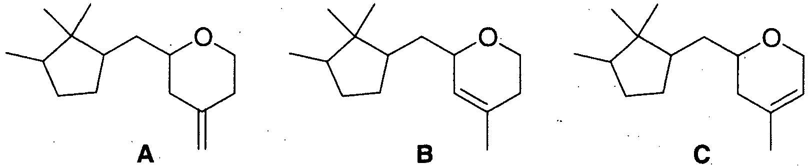

A mixture of isomers (A), (B) and (C) in a 63:14:23 ratio was prepared, according to example 1, starting from hydrogenated campholenaldehyde.

Its odour is similar to that of example 1.

### Main isomers (A):

¹H-NMR (CDCl₃, 200MHz): δ (ppm) 0.45-0.55 (m, 3H), 0.75-0.85 (m, 6H), 1.0-1.3 (m, 2H), 1.3-1.55 (m, 3H), 1.55-2.0 (m, 5H), 2.0-2.4 (m, 2H), 3.1-3.4 (m, 2H), 3.9-4.05 (m, 1H), 4.65-4.75 (m, 2H).
¹³C-NMR (CDCl₃, 60MHz) : δ (ppm) 13.89 & 14.23, 25.34 & 25.46, 28.04 & 28.43, 30.11 & 30.24, 35.22 & 35.27, 37.20, 40.81 & 42.24, 44.87 & 44.95, 46.05 & 47.07, 46.14 & 46.19, 68.55 & 68.64, 77.63 & 78.54, 108.01 & 108.17, 144.75 & 1.44.97.
**(B)** Isomers (*Characteristic protons*): δ (ppm) 3.9-4.05 (m, 3H), 5.25-5.30 (m, 1H).
¹³C-NMR (CDCl₃, 60MHz, selected data): δ (ppm) 63.12 & 63.44, 72.53 & 72.72, 123.67 & 125.10.
**(C)** Isomers (*Characteristic protons*): δ (ppm) 3.4-3.65 (m, 1H), 3.95-4.15 (m, 2H).
¹³C-NMR (CDCl₃, 60MHz, selected data): δ (ppm) 65.76 & 65.84, 73.39 & 73.72, 119.65 & 119.74.

### Example 4: 4-methyl-4-(3-methyl-but-3-enyloxy)-2-(2,2,3-trimethyl-cyclo-pentylmethyl)-tetrahydropyran

4-Methyl-4-(3-methyl-but-3-enyloxy)-2-(2,2,3-trimethyl-cyclopentylmethyl)-tetra-hydropyran was obtained under the conditions used in example 3 and was separated from the other derivatives by fine fractionation.

It consists in a *trans*/*cis* (60:40) mixture of isomers.

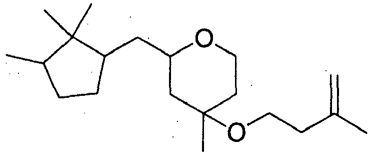

Its odour is not powerful, bitter orange.
Major diastereoisomers, trans isomers:
¹H-NMR (CDCl₃, 200MHz): δ (ppm) 0.49 (s, 3H), 0.82 (d, 3H, *J* = 10.1Hz), 0.83 (s, 3H), 1.0-1.9 (m, 12H), 1.28 (s, 3H), 1.74 (s, 3H), 2.24 (t, 2H, *J* = 7.1Hz), 3.2-3.5 (m, 2H), 3.50 (t, 2H, *J* = 7.2Hz), 4.74 (m, 2H).
¹³C-NMR (CDCl₃ 60MHz): δ (ppm) 13.91 & 14.29, 21.12 & 21.34, 23.0, 25.38 & 25.46, 28.08 & 28.25, 30.11. & 30.23, 37.24 & 37.35, 37.4 & 37.49, 38.65, 42.08 & 42.39, 43.11 & 44.41, 44.88 & 44.98, 46.11 & 47.0, 58.95, 65.05 & 65.13, 72.60 & 72.66, 73.79 & 74.74, 111.15, 143.24.
MS [e/m (%)]: 308(M+, 1), 293(2), 223(23), 109(10), 99(72), 97(10), 69(100), 55(10), 43(11), 41(36).
IR (film, cm⁻¹): 642w, 887m, 1078m, 1104s, 1144m, 1177m, 1251w, 1271w, 1374m, 1453m, 1466m, 1650w, 2869s, 2951s.

### Minor diastereoisomers, cis isomers:

¹H-NMR (CDCl₃, 200MHz), selected data: δ (ppm) 0.48 (s, 3H), 1.27 (s, 3H), 3.49 (t, 2H, J = 7.2Hz).
¹³C-NMR (CDCl₃, 60MHz), selected data: δ (ppm) 111.36, 143.39.
MS [e/m (%)]: 308(M+, 1), 293(9), 223(11), 222(11), 221(37), 112(25), 109(12), 99(26), 97(28), 95(12), 69(100), 55(16), 43(17), 41(50).
IR (film, cm⁻¹): 642w, 887m, 1078m, 1104s, 1144m, 1177m, 1251w, 1271w, 1374m, 1453m, 1466m, 1650w, 2869s, 2951s.

### Example 5: Fragrance composition containing the (A)/(B)/(C) mixture obtained in example 1

A base perfuming composition was prepared as described from the following ingredients:

| | |
|---|---|
| ABRAC NERYL ACETATE | 5 |
| BENZYL ACETATE | 5 |
| BERGAMYL ACETATE | 70 |
| GERANYL ACETATE | 7 |
| LINALYL ACETATE | 45 |
| TERPENYL ACETATE | 60 |
| ACETYL ISOEUGENOL | 5 |
| PHENYLETHYLALCOHOL | 130 |
| BENZALDEHYDE | 2 |
| ALDEHYDE C10 | 2 |
| CYCLAMEN ALDEHYDE^{®} | 25 |
| ANISALDEHYDE | 20 |
| VELVIONE^{®} | 5 |
| GLOBANONE^{®} | 5 |
| METHYL BENZOATE | 2 |
| CETONE MIEL | 5 |
| ETHYL LINALOL | 100 |
| FLOROL^{®} | 45 |
| BOIS DE GAIAC ESS. | 2 |
| METHYL DIHYDROJASMONATE | 40 |
| IONONE ALPHA | 30 |
| IONONE BETA | 10 |
| MAYOL^{®} | 30 |
| METHYL ISOEUGENOL | 2 |
| NEROLINE^{®} | 15 |
| LINALOL OXIDE | 3 |
| TERPINEOL | 85 |
| TETRAHYDROLINALOL | 30 |
| VANILLINE | 1 |
| ACETOPHENONE 10% DPG | 5 |
| PHENYLACETIC ACID 10% DPG | 2 |
| CITRONELLAL C 10% DPG. | 2 |
| INDOLAROME^{®} 10% DPG | 5 |
| | 800 |

To this base composition of the honeysuckle type were added 10 parts in weight of the (A)/(B)/(C) mixture of isomers obtained in example 1. The novel composition thus obtained showed a greener note, reminding petit-grain essence, with a light orange blossom undertone. Furthermore, the (A)/(B)/(C) mixture imparts freshness to the composition, those interesting aspects of the new fragrance composition have been appreciated in alcoholic, as well as in a shower gel and in a cream base.

## Claims

1. Pyran derivatives of general formula (I) 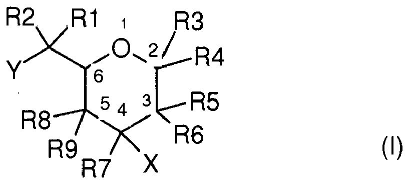 wherein
• Y = 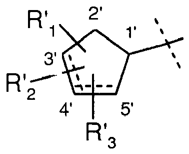 wherein R'1, R'2 and R'3 are each independently a hydrogen atom or a methyl group or an ethyl group, at least one of R'1, R'2 and R'3 being a methyl or an ethyl group,
wherein the dotted lines represent a double bond and is present or not, and
• R1, R2, R3, R4 are, each independently, a hydrogen atom or a linear or branched C₁₋₅ alkyl or C₂₋₅ alkenyl group, and
• X is present or absent,
• when X is present,
o R5, R6, R7, R8, R9 are all present, and X is a hydrogen atom or an OZ group, wherein Z is a hydrogen atom or a R10 group or a C(O)R10 group
• when X is absent, a double bond involving the carbon atom at the 4 position is present and
o R7, R8 and R9 are present, and one of R5 or R6 is present and the other one is absent, or
o R5, R6, and R7 are present, and one of R8 or R9 is present and the other one is absent, or
o R7 is a =C(R11)(R12) group and R5, R6, R8, R9 are present,
• and when they are present, each of R5-R12 group is, independently, a hydrogen atom or a linear or branched C₁₅ alkyl or C₂₋₅ alkenyl group.

2. Pyran derivatives of general formula (I) according to Claim **1** wherein
• Y is one of (Y1), (Y2), (Y3) or (Y4) group 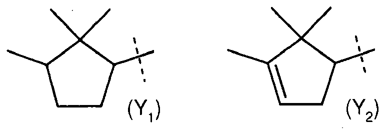 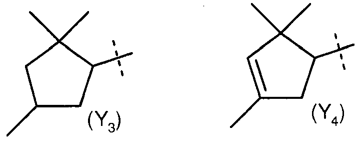
• R1, R2, R3, R4 are, each independently, a hydrogen atom, a linear or branched C₁₋₅ alkyl or C₂₋₅ alkenyl group, and
• X is present or absent,
• when X is present,
o R5, R6, R7, R8, R9 are all present, and X is a hydrogen atom or an OZ group, wherein Z is a hydrogen atom or a R10 group or a C(O)R10 group
• when X is absent, a double bond involving the carbon atom at the 4 position is present and
o R7, R8 and R9 are present, and one of R5 or R6 is present and the other one is absent, or
o R5, R6, and R7 are present, and one of R8 or R9 is present and the other one is absent, or
o R7 is a =C(R11)(R12) group and R5, R6, R8, R9 are present,
• and when they are present, each of R5-R12 group is, independently, a hydrogen atom, a linear or branched C₁₋₅ alkyl or C₂₋₅ alkenyl group.

3. Pyran derivatives of general formula (I) according to Claims **1 or 2,** wherein
• Y is as defined in claims **1** or **2,**
• one of R3 or R4 is a hydrogen atom, and the other one is a methyl group
• R7 is a =CH2 group and X is absent; or
• R7 is a methyl group and X is present or absent ; when R7 is a methyl group and X is present, X is a hydrogen atom or an OZ group, wherein Z is a hydrogen atom or a R10 group or a C(O)R10 group; when R7 is a methyl group and X is absent, a double bond involving the carbon atom at the 4 position is present and
o R8 and R9 are present, and one of R5 or R6 is present and the other one is absent, or
o R5, R6, are present, and one of R8 or R9 is present and the other one is absent,
• R1, R2 and R10 are, each independently, a hydrogen atom, or a linear or branched C₁₋₅ alkyl or C₂₋₅ alkenyl group
• when present, R5, R6, R8, R9 are each a hydrogen atom.

4. Pyran derivatives of general formula (I) according to Claim **1,** wherein said derivatives are selected in the group comprising 4-methylene-2-(2,2,3-trimethyl-cyclopent-3-enylmethyl)-tetrahydropyran, 4-methyl-6-(2,2,3-trimethyl-cyclopent-3-enylmethyl)-3,6-dihydro-2H-pyran, 4-methyl-2-(2,2,3-trimethyl-cyclopent-3-enylmethyl)-3,6-dihydro-2H-pyran, 4-methyl-2-(2,2,3-trimethyl-cyclopentylmethyl)-tetrahydropyran, 4-methylene-2-(2,2,3-trimethylcyclopentylmethyl)-tetrahydropyran, 4-methyl-6-(2,2,3-trimethyl-cyclopentylmethyl)-3,6-dihydro-2H-pyran, 4-methyl-2-(2,2,3-trimethyl-cyclopentylmethyl)-3,6-dihydro-2H-pyran, 4-methyl-4-(3-methyl-but-3-enyloxy)-2-(2,2,3-trimethyl-cyclopentylmethyl)-tetrahydropyran.

5. A process for the preparation of pyran derivatives of formula (I) as described in anyone of claims **1** to **4,** said process being **characterized in that** a compound of general formula (A) 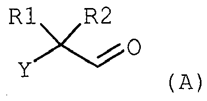 wherein
• Y is as defined in claim **1**,
• R1 and R2 are, each independently, a hydrogen atom or a linear or branched C₁₋₅ alkyl or C₂₋₅alkenyl group,
is reacted, in presence of an acid, with a compound of general formula (B) 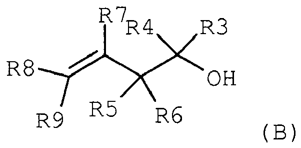 wherein
R3, R4, R5, R6, R7, R8 and R9 are, each independently, a hydrogen atom or a linear or branched C₁₋₅ alkyl group, or a linear or branched C₂₋₅ alkenyl group.

6. The process as claimed in claim **5,** further comprising a step to convert the aldehyde (A) into an acetal of general formula (C) 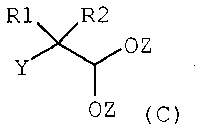 wherein
• Y is as defined in claim **1,**
• R1, R2, are, each independently, a hydrogen atom, a linear or branched C₁₋₅ alkyl or C₂₋₅ alkenyl group,
• Z is a R10 group wherein R10 is a linear or branched C₁₋₅ alkyl or C₂₋₅ alkenyl group,
prior to the reaction with the alcohol (B).

7. The process as claimed in claims **5** and **8,** wherein compound (A) or compound (C) is reacted with compound (B), in presence of an acid, to produce the compounds of formula (I) wherein X is OZ, Z being a hydrogen atom or a linear or branched C₁₋₅ alkyl or C₂₋₅ alkenyl group.

8. The process as claimed in claim **5,** wherein compound (A) is reacted with compound (B), in presence of an acid, and the compounds of formula (I), wherein X is OH, are separated from the compounds of formula (I) wherein X is absent, and then subjected to an esterification reaction, in order to obtain compounds of formula (I) wherein X is OC(O)R10.

9. The process as claimed in Claim **5,** wherein compound (A) is reacted with compound (B), in presence of an acid, and the compounds of formula (I), wherein X is OH, are separated from the compounds of formula (I) wherein X is absent and then subjected to an alkylation reaction in order to obtain compounds of formula (I) wherein X is OZ, Z being a linear or branched C₁₋₅ alkyl or C₂₋₅ alkenyl group.

10. Composition comprising at least one compound of formula (II), and/or at least one compound of general formula (III) and/or at least one compound of general formula (IV), and optionally (V) and/or (VI) 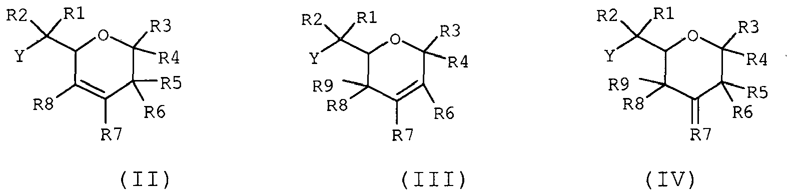 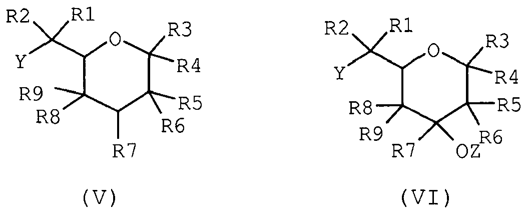 wherein
• Y is 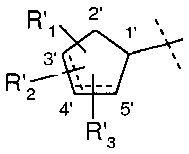 wherein R'1, R'2 and R'3 are each independently a hydrogen atom or a methyl group or an ethyl group, at least one of R'1, R'2 and R'3 being a methyl or an ethyl group,
wherein the dotted lines represent a double bond and is present or not,
• R1, R2, R3, R4, R5, R6, R7, R8, R9 are, each independently, a hydrogen atom, a linear or branched C₁₋₅ alkyl or C₂₋₅ alkenyl group,
• Z is a hydrogen atom or a linear or branched C₁₋₅ alkyl or C₂₋₅ alkenyl group or a group C(O)R10 wherein R10 is a linear or branched C₁₋₅ alkyl or C₂₋₅ alkenyl group.

11. Use of a pyran derivative according to anyone of claims **1** to **4,** or of a composition according to Claim **10** in perfumery for the preparation of perfumed bases and concentrates, fragrances, perfumes; topic compositions; cosmetic compositions selected in the group comprising face and body creams, cleansers, facial treatments, talc powders, hair oils, shampoos, hair lotions, bath oils and salts, shower and bath gels, soaps, body anti-perspirants and deodorizers, pre-shave, shaving and post-shave creams and lotions, creams, toothpastes, mouth baths, pomades; cleaning products, selected in the group comprising softeners, detergents, air deodorizers and household cleaning supplies.

12. Use of a pyran derivative according to anyone of claims **1** to **4,** or of a composition according to Claim **10,** as flavouring agents for the preparation of flavouring compositions or articles, selected in the group comprising drinks, dairy products, ice creams, soups, sauces, dips, dishes, meat products, culinary assistances, salted biscuits or snacks and also beers, wines and tobaccos.

13. Use of a pyran derivative according to anyone of claims **1** to **4,** or of a composition according to Claim **10,** as masking agents of odours and/or flavours, in a pharmaceutical, cosmetic or food composition.

14. Use of a pyran derivative according to anyone of claims **1** to **4,** or of a composition according to Claim **10,** in combination with other perfumed or flavoured ingredients, solvents, additives or fixatives.

## Patentansprüche

1. Pyranderivate der allgemeinen Formel (I) 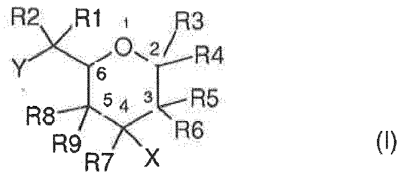 worin
• Y = 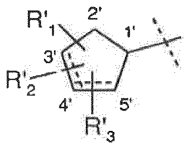 worin R`1, R'2 und R'3 jeweils unabhängig voneinander für ein Wasserstoffatom oder eine Methylgruppe oder eine Ethylgruppe stehen, wobei mindestens eine der Variablen R`1, R'2 und R'3 für eine Methyl- oder Ethylgruppe steht,
wobei die gestrichelten Linien eine Doppelbindung darstellen, die vorhanden sein kann oder nicht, und
• R1, R2, R3 und R4 jeweils unabhängig voneinander für ein Wasserstoffatom oder eine lineare oder verzweigte C₁₋₅-Alkyl- oder C₂₋₅-Alkenylgruppe stehen und
• X vorhanden ist oder fehlt,
• wenn X vorhanden ist,
o R5, R6, R7, R8 und R9 alle vorhanden sind und X für ein Wasserstoffatom oder eine OZ-Gruppe steht, wobei Z für ein Wasserstoffatom oder eine R10-Gruppe oder eine C(O)R10-Gruppe steht,
• wenn X fehlt, eine Doppelbindung unter Beteiligung des Kohlenstoffatoms in der 4-Stellung vorhanden ist und
o R7, R8 und R9 vorhanden sind und entweder R5 oder R6 vorhanden ist und die andere fehlt, oder
o R5, R6 und R7 vorhanden sind und entweder R8 oder R9 vorhanden ist und die andere fehlt, oder
o R7 für eine =C(R11)(R12)-Gruppe steht und R5, R6, R8 und R9 vorhanden sind,
• und dann, wenn sie vorhanden sind, jede der Gruppen R5-R12 unabhängig voneinander für ein Wasserstoffatom oder eine lineare oder verzweigte C₁₋₅-Alkyl- oder C₂₋₅-Alkenylgruppe stehen.

2. Pyranderivate der allgemeinen Formel (I) nach Anspruch 1, wobei
• Y für eine Gruppe (Y1), (Y2), (Y3) oder (Y4) steht 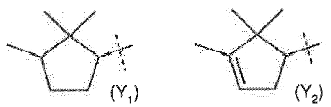 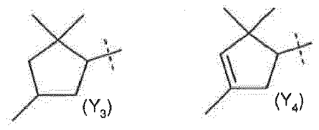
• R1, R2, R3 und R4 jeweils unabhängig voneinander für ein Wasserstoffatom oder eine lineare oder verzweigte C₁₋₅-Alkyl- oder C₂₋₅-Alkenylgruppe stehen und
• X vorhanden ist oder fehlt,
• wenn X vorhanden ist,
o R5, R6, R7, R8 und R9 alle vorhanden sind und X für ein Wasserstoffatom oder eine OZ-Gruppe steht, wobei Z für ein Wasserstoffatom oder eine R10-Gruppe oder eine C(O)R10-Gruppe steht,
• wenn X fehlt, eine Doppelbindung unter Beteiligung des Kohlenstoffatoms in der 4-Stellung vorhanden ist und
o R7, R8 und R9 vorhanden sind und entweder R5 oder R6 vorhanden ist und die andere fehlt, oder
o R5, R6 und R7 vorhanden sind und entweder R8 oder R9 vorhanden ist und die andere fehlt, oder
o R7 für eine =C(R11)(R12)-Gruppe steht und R5, R6, R8 und R9 vorhanden sind,
• und dann, wenn sie vorhanden sind, jede der Gruppen R5-R12 unabhängig voneinander für ein Wasserstoffatom oder eine lineare oder verzweigte C₁₋₅-Alkyl- oder C₂₋₅-Alkenylgruppe stehen.

3. Pyranderivate der allgemeinen Formel (I) nach Anspruch 1 oder 2, wobei
• Y wie in Anspruch 1 oder 2 definiert ist,
• eine der Variablen R3 oder R4 für ein Wasserstoffatom steht und die andere für eine Methylgruppe steht,
• R7 für eine =CH2-Gruppe steht und X fehlt; oder
• R7 für eine Methylgruppe steht und X vorhanden ist oder fehlt; dann,
wenn R7 für eine Methylgruppe steht und X vorhanden ist, X für ein Wasserstoffatom oder eine OZ-Gruppe steht, wobei Z für ein Wasserstoffatom oder eine R10-Gruppe oder eine C(O)R10-Gruppe steht; dann, wenn R7 für eine Methylgruppe steht und X fehlt, eine Doppelbindung unter Beteiligung des Kohlenstoffatoms in der 4-Stellung vorhanden ist und
o R8 und R9 vorhanden sind und entweder R5 oder R6 vorhanden ist und die andere fehlt, oder
o R5 und R6 vorhanden sind und entweder R8 oder R9 vorhanden ist und die andere fehlt,
• R1, R2 und R10 jeweils unabhängig voneinander für ein Wasserstoffatom oder eine lineare oder verzweigte C₁₋₅-Alkyl- oder C₂₋₅-Alkenylgruppe stehen,
• sofern vorhanden, R5, R6, R8 und R9 jeweils für ein Wasserstoffatom stehen.

4. Pyranderivate der allgemeinen Formel (I) nach Anspruch 1, wobei die Derivate aus der Gruppe umfassend 4-Methylen-2-(2,2,3-trimethyl-cyclopent-3-enylmethyl)-tetrahydropyran, 4-Methyl-6-(2,2,3-trimethyl-cyclopent-3-enylmethyl)-3,6-dihydro-2H-pyran, 4-Methyl-2-(2,2,3-trimethyl-cyclopent-3-enylmethyl)-3,6-dihydro-2H-pyran, 4-Methyl-2-(2,2,3-trimethyl-cyclopentylmethyl)-tetrahydropyran, 4-Methylen-2-(2,2,3-trimethyl-cyclopentylmethyl)-tetrahydropyran, 4-Methyl-6-(2,2,3-trimethyl-cyclopentylmethyl)-3,6-dihydro-2H-pyran, 4-Methyl-2-(2,2,3-trimethyl-cyclopentylmethyl)-3,6-dihydro-2H-pyran und 4-Methyl-4-(3-methylbut-3-enyloxy)-2-(2,2,3-trimethyl-cyclopentylmethyl)-tetrahydropyran ausgewählt sind.

5. Verfahren zur Herstellung von Pyranderivaten der Formel (I) gemäß einem der Ansprüche 1 bis 4, wobei das Verfahren **dadurch gekennzeichnet ist, dass** man eine Verbindung der allgemeinen Formel (A) 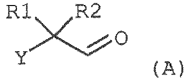 worin
• Y wie in Anspruch 1 definiert ist,
• R1 und R2 jeweils unabhängig voneinander für ein Wasserstoffatom oder eine lineare oder verzweigte C₁₋₅-Alkyl- oder C₂₋₅-Alkenylgruppe stehen,
in Gegenwart einer Säure mit einer Verbindung der allgemeinen Formel (B) 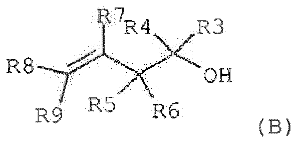
worin
R3, R4, R5, R6, R7, R8 und R9 jeweils unabhängig voneinander für ein Wasserstoffatom oder eine lineare oder verzweigte C₁₋₅-Alkylgruppe oder eine lineare oder verzweigte C₂₋₅-Alkenylgruppe stehen,
umsetzt.

6. Verfahren nach Anspruch 5, bei dem man ferner den Aldehyd (A) vor der Umsetzung mit dem Alkohol (B) in ein Acetal der allgemeinen Formel (C) 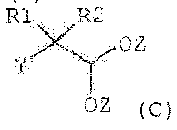 worin
• Y wie in Anspruch 1 definiert ist,
• R1 und R2 jeweils unabhängig voneinander für ein Wasserstoffatom oder eine lineare oder verzweigte C₁₋₅-Alkyl- oder C₂₋₅-Alkenylgruppe stehen,
• Z für eine R10-Gruppe steht, wobei R10 für eine lineare oder verzweigte C₁₋₅-Alkyl- oder C₂₋₅-Alkenylgruppe steht,
umwandelt.

7. Verfahren nach den Ansprüchen 5 und 6, bei dem man Verbindung (A) oder Verbindung (C) in Gegenwart einer Säure mit Verbindung (B) zu Verbindungen der Formel (I), worin X für OZ steht, wobei Z für ein Wasserstoffatom oder eine lineare oder verzweigte C₁₋₅-Alkyl- oder C₂₋₅-Alkenylgruppe steht, umsetzt.

8. Verfahren nach Anspruch 5, bei dem man Verbindung (A) in Gegenwart einer Säure mit Verbindung (B) umsetzt und die Verbindungen der Formel (I), worin X für OH steht, von den Verbindungen der Formel (I), worin X fehlt, abtrennt und dann einer Veresterungsreaktion unterwirft, um Verbindungen der Formel (I), worin X für OC(O)R10 steht, zu erhalten.

9. Verfahren nach Anspruch 5, bei dem man Verbindung (A) in Gegenwart einer Säure mit Verbindung (B) umsetzt und die Verbindungen der Formel (I), worin X für OH steht, von den Verbindungen der Formel (I), worin X fehlt, abtrennt und dann einer Alkylierungsreaktion unterwirft, um Verbindungen der Formel (I), worin X für OZ steht, wobei Z für eine lineare oder verzweigte C₁₋₅-Alkyl- oder C₂₋₅-Alkenylgruppe steht, zu erhalten.

10. Zusammensetzung, umfassend mindestens eine Verbindung der Formel (II) und/oder mindestens eine Verbindung der allgemeinen Formel (III) und/oder mindestens eine Verbindung der allgemeinen Formel (IV) und optional(V) und/oder (VI) 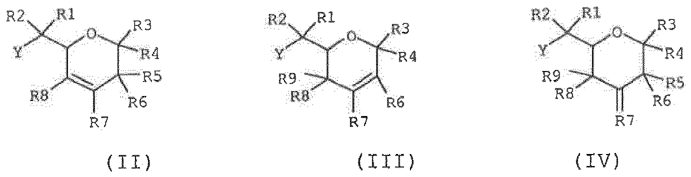 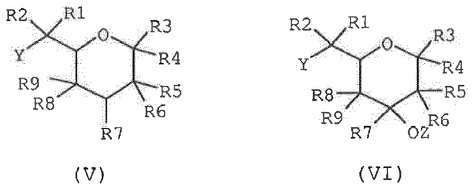 worin
• Y für 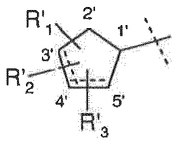 steht, worin R`1, R'2 und R'3 jeweils unabhängig voneinander für ein Wasserstoffatom oder eine Methylgruppe oder eine Ethylgruppe stehen, wobei mindestens eine aus R`1, R'2 und R'3 für eine Methyl- oder Ethylgruppe steht,
wobei die gestrichelten Linien eine Doppelbindung darstellen, die vorhanden sein kann oder nicht, und
• R1, R2, R3, R4, R5, R6, R7, R8 und R9 jeweils unabhängig voneinander für ein Wasserstoffatom oder eine lineare oder verzweigte C₁₋₅-Alkyl- oder C₂₋₅-Alkenylgruppe stehen,
• Z für ein Wasserstoffatom oder eine lineare oder verzweigte C₁₋₅-Alkyl- oder C₂₋₅-Alkenylgruppe oder eine C(O)R10-Gruppe steht, wobei R10 für eine lineare oder verzweigte C₁₋₅-Alkyl- oder C₂₋₅-Alkenylgruppe steht.

11. Verwendung eines Pyranderivats nach einem der Ansprüche 1 bis 4 oder einer Zusammensetzung nach Anspruch 10 in der Parfümerie zur Herstellung von parfümierten Basen und Konzentraten, Duftstoffen, Parfümen; topischen Zusammensetzungen; kosmetischen Zusammensetzungen aus der Gruppe umfassend Gesichts- und Körpercremes, Reinigern, Gesichtsbehandlungen, Talkumpudern, Haarölen, Shampoos, Haarlotionen, Badeölen und -salzen, Dusch- und Badegelen, Seifen, Körperantitranspirantien und -desodorantien, Preshave-, Rasier- und Aftershave-Cremes und -Lotionen, Cremes, Zahnpasten, Mundspülungen, Pomaden; Reinigungsprodukten, ausgewählt aus der Gruppe umfassend Weichspüler, Wasch- und Reinigungsmittel, Luftdesodorantien und Haushaltsreinigungsbedarf.

12. Verwendung eines Pyranderivats nach einem der Ansprüche 1 bis 4 oder einer Zusammensetzung nach Anspruch 10 als Geschmacksstoff zur Herstellung von Geschmackszusammensetzungen oder -artikeln, ausgewählt aus der Gruppe umfassend Getränke, Molkereiprodukte, Eiscremes, Suppen, Soßen, Dips, Gerichten, Fleischprodukte, kulinarische Hilfsstoffe, Salzgebäck oder Snacks sowie Biere, Weine und Tabake.

13. Verwendung eines Pyranderivats nach einem der Ansprüche 1 bis 4 oder einer Zusammensetzung nach Anspruch 10 als Geruchs- und/oder Geschmacksmaskierungsmittel in einer pharmazeutischen Zusammensetzung, kosmetischen Zusammensetzung oder Lebensmittelzusammensetzung.

14. Verwendung eines Pyranderivats nach einem der Ansprüche 1 bis 4 oder einer Zusammensetzung nach Anspruch 10 in Kombination mit anderen parfümierten oder aromatisierten Bestandteilen, Lösungsmitteln, Additiven oder Fixierungsmitteln.

## Revendications

1. Dérivés de pyrane de formule générale (I) 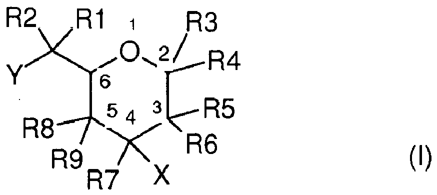 dans laquelle
Y = 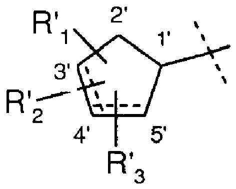 dans laquelle R'1, R'2 et R'3 sont chacun indépendamment un atome d'hydrogène ou un groupe méthyle ou un groupe éthyle, au moins un des R'1, R'2 et R'3 étant un groupe méthyle ou éthyle,
dans laquelle les lignes pointillées représentent une liaison double et est présente ou non, et
R1, R2, R3, R4 sont, chacun indépendamment, un atome d'hydrogène ou un groupe alcényle en C₂ à C₅ ou alkyle en C₁ à C₅ linéaire ou ramifié, et
X est présent ou absent,
quand X est présent,
- R5, R6, R7, R8, R9 sont tous présents, et X est un atome d'hydrogène ou un groupe OZ, dans lequel Z est un atome d'hydrogène ou un groupe R10 ou un groupe C (O) R10
quand X est absent, une liaison double mettant en jeu l'atome de carbone en position 4 est présente et
- R7, R8 et R9 sont présents, et un des R5 ou R6 est présent et l'autre est absent, ou
- R5, R6 et R7 sont présents, et un des R8 ou R9 est présent et l'autre est absent, ou
- R7 est un groupe =C(R11)(R12) et R5, R6, R8, R9 sont présents,
et quand ils sont présents, chacun des groupes R5 à R12 est, indépendamment, un atome d'hydrogène ou un groupe alcényle en C₂ à C₅ ou alkyle en C₁ à C₅ linéaire ou ramifié

2. Dérivés de pyrane de formule générale (I) selon la revendication 1 dans laquelle
Y est un des groupes (Y1), (Y2), (Y3) ou Y(4) 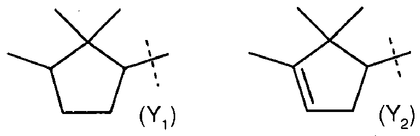 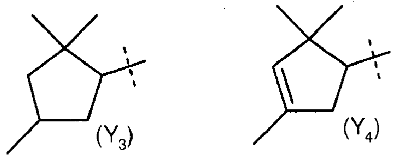
R1, R2, R3, R4 sont, chacun indépendamment, un atome d'hydrogène, ou un groupe alcényle en C₂ à C₅ ou alkyle en C₁ à C₅ linéaire ou ramifié, et
X est présent ou absent,
quand X est présent,
- R5, R6, R7, R8, R9 sont tous présents, et X est un atome d'hydrogène ou un groupe OZ, dans lequel Z est un atome d'hydrogène ou un groupe R10 ou un groupe C(O)R10
quand X est absent, une liaison double mettant en jeu l'atome de carbone en position 4 est présente et
- R7, R8 et R9 sont présents, et un des R5 ou R6 est présent et l'autre est absent, ou
- R5, R6 et R7 sont présents, et un des R8 ou R9 est présent et l'autre est absent, ou
- R7 est un groupe =C(R11) (R12) et R5, R6, R8, R9 sont présents,
et quand ils sont présents, chacun des groupes R5 à R12, est indépendamment, un atome d'hydrogène ou un groupe alcényle en C₂ à C₅ ou alkyle en C₁ à C₅ linéaire ou ramifié.

3. Dérivés de pyrane de formule générale (I) selon la revendication 1 ou 2, dans laquelle
Y est tel que défini dans la revendication 1 ou 2,
un des R3 ou R4 est un atome d'hydrogène, et l'autre est un groupe méthyle R7 est un groupe =CH2 et X est absent ; ou
R7 est un groupe méthyle et X est présent ou absent ; quand R7 est un groupe méthyle et X est présent, X est un atome d'hydrogène ou un groupe OZ, dans lequel Z est un atome d'hydrogène ou un groupe R10 ou un groupe C(O)R10 ; quand R7 est un groupe méthyle et X est absent, une liaison double mettant en jeu l'atome de carbone en position 4 est présente et
- R8 et R9 sont présents, et un des R5 ou R6 est présent et l'autre est absent, ou
- R5, R6, sont présents, et un des R8 ou R9 est présent et l'autre est absent,
R1, R2 et R10 sont, chacun indépendamment, un atome d'hydrogène, ou un groupe alcényle en C₂ à C₅ ou alkyle en C₁ à C₅ linéaire ou ramifié
quand ils sont présents, R5, R6, R8, R9 sont chacun un atome d'hydrogène.

4. Dérivés de pyrane de formule générale (I) selon la revendication 1, dans laquelle lesdits dérivés sont choisis parmi le groupe constitué du 4-méthylène-2-(2,2,3-triméthyl-cyclopent-3-ènylméthyl)-tétrahydropyrane, 4-méthyl-6-(2,2,3-triméthylcyclopent-3-ènylméthyl)-3,6-dihydro-2H-pyrane, 4-méthyl-2-(2,2,3-triméthylcyclopent-3-ènylméthyl)-3,6-dihydro-2H-pyrane, 4-méthyl-2-(2,2,3-triméthylcyclopentylméthyl)-tétrahydropyrane, 4-méthylène-2-(2,2,3-triméthylcyclopentylméthyl)-tétrahydropyrane, 4-méthyl-6-(2,2,3-triméthylcyclopentylméthyl)-3,6-dihydro-2H-pyrane, 4-méthyl-2-(2,2,3-triméthylcyclopentylméthyl)-3,6-dihydro-2H-pyrane, 4-méthyl-4-(3-méthyl-but-3-ènyloxy)-2-(2,2,3-triméthyl-cyclopentylméthyl)-tétrahydropyrane.

5. Procédé de préparation de dérivés de pyrane de formule (I) telle que décrite dans l'une quelconque des revendications 1 à 4, ledit procédé étant **caractérisé en ce qu'**un composé de formule générale (A) 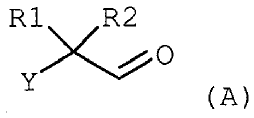
dans laquelle
Y est tel que défini dans la revendication 1,
R1 et R2 sont, chacun indépendamment, un atome d'hydrogène ou un groupe alkyle en C₁ à C₅ linéaire ou ramifié ou alcényle en C₂ à C₅,
est mis à réagir, en présence d'un acide, avec un composé de formule générale (B) 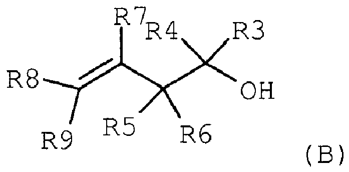 dans laquelle
R3, R4, R5, R6, R7, R8 et R9 sont, chacun indépendamment, un atome d'hydrogène ou un groupe alcényle en C₂ à C₅ ou alkyle en C₁ à C₅ linéaire ou ramifié.

6. Procédé selon la revendication 5, comportant en outre une étape consistant à convertir l'aldéhyde (A) en un acétal de formule générale (C) 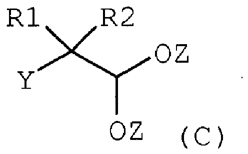 dans laquelle
Y est tel que défini dans la revendication 1,
R1, R2, sont, chacun indépendamment, un atome d'hydrogène, un groupe alcényle en C₂ à C₅ ou alkyle en C₁ à C₅ linéaire ou ramifié,
Z est un groupe R10 dans lequel R10 est un groupe alcényle en C₂ à C₅ ou alkyle en C₁ à C₅ linéaire ou ramifié,
avant la réaction avec l'alcool (B).

7. Procédé selon les revendications 5 et 8, dans lequel un composé (A) ou un composé (C) est mis à réagir avec un composé (B), en présence d'un acide, pour produire les composés de formule (I) dans laquelle X est OZ, Z étant un atome d'hydrogène ou un groupe alcényle en C₂ à C₅ ou alkyle en C₁ à C₅ linéaire ou ramifié.

8. Procédé selon la revendication 5, dans lequel un composé (A) est mis à réagir avec un composé (B), en présence d'un acide, et les composés de formule (I), dans laquelle X est OH, sont séparés des composés de formule (I) dans laquelle X est absent, et ensuite soumis à une réaction d'estérification, afin d'obtenir des composés de formule (I) dans laquelle X est OC(O)R10.

9. Procédé selon la revendication 5, dans lequel un composé (A) est mis à réagir avec un composé (B), en présence d'un acide, et les composés de formule (I), dans laquelle X est OH, sont séparés des composés de formule (I) dans laquelle X est absent et ensuite soumis à une réaction d'alkylation afin d'obtenir des composés de formule (I) dans laquelle X est OZ, Z étant un groupe alcényle en C₂ à C₅ ou alkyle en C₁ à C₅ linéaire ou ramifié.

10. Composition comprenant au moins un des composés de formule (II), et/ou au moins un composé de formule générale (III) et/ou au moins un composé de formule générale (IV), et éventuellement (V) et/ou (VI) 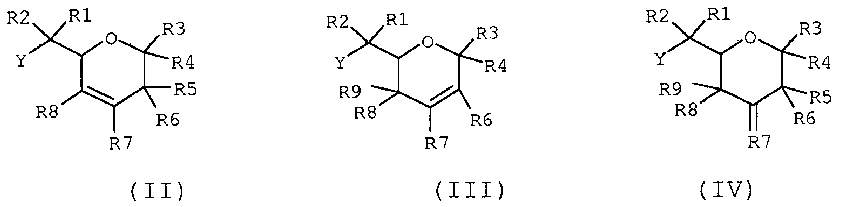 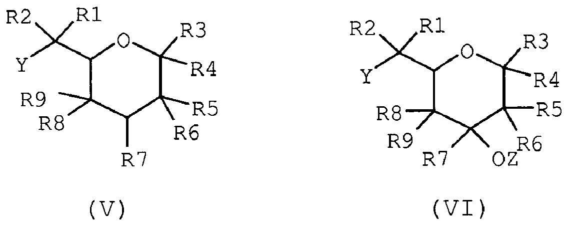 dans lesquelles
Y est 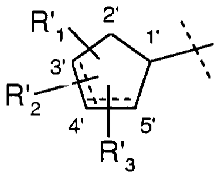 dans laquelle R'1, R'2 et R'3 sont chacun indépendamment un atome d'hydrogène ou un groupe méthyle ou un groupe éthyle, au moins un des R'1, R'2 et R'3 étant un groupe méthyle ou éthyle,
dans laquelle les lignes pointillées représentent une liaison double et est présente ou non,
R1, R2, R3, R4, R5, R6, R7, R8, R9 sont, chacun indépendamment, un atome d'hydrogène, un groupe alcényle en C₂ à C₅ ou alkyle en C₁ à C₅ linéaire ou ramifié,
Z est un atome d'hydrogène ou un groupe alcényle en C₂ à C₅ ou alkyle en C₁ à C₅ linéaire ou ramifié ou un groupe C(O)R10 dans lequel R10 est un groupe alcényle en C₂ à C₅ ou alkyle en C₁ à C₅ linéaire ou ramifié.

11. Utilisation d'un dérivé de pyrane selon l'une quelconque des revendications 1 à 4, ou d'une composition selon la revendication 10 en parfumerie pour la préparation de bases et concentrés parfumés, de fragrances et parfums ; de compositions topiques ; de compositions cosmétiques sélectionnées dans le groupe constitué de crèmes pour le visage et pour le corps ; de nettoyants, de traitements faciaux, de poudres de talc, d'huiles capillaires, de shampoings, de lotions capillaires, d'huiles et de sels pour le bain, de gels pour la douche et le bain, de savons, d'anti-transpirants et de déodorants corporels, de crèmes et de lotions pour le prérasage, le rasage et l'après rasage, de crèmes, de dentifrices, de bains pour la bouche, de pommades ; de produits de nettoyage sélectionnés dans le groupe constitué d'adoucissants, de détergents, de désodorisants pour l'air et de nettoyants ménagers.

12. Utilisation d'un dérivé de pyrane selon l'une quelconque des revendications 1 à 4, ou d'une composition selon la revendication 10, en tant qu'agents aromatisants pour la préparation de compositions ou d'articles aromatisants, sélectionnés dans le groupe constitué de boissons, de produits laitiers, de glaces, de soupes, de sauces, de sauces amuse-gueules, de plats, de préparations à base de viande, d'aides culinaires, de biscuits ou d'en-cas salés et également de bières, de vins et de tabacs.

13. Utilisation d'un dérivé de pyrane selon l'une quelconque des revendications 1 à 4, ou d'une composition selon la revendication 10, en tant qu'agents masquants d'odeurs et/ou d'arômes, dans une composition pharmaceutique, cosmétique ou alimentaire.

14. Utilisation d'un dérivé de pyrane selon l'une quelconque des revendications 1 à 4, ou d'une composition selon la revendication 10, en association avec d'autres ingrédients parfumés ou aromatisés, solvants, additifs ou produits fixateurs.
